# EUROPEAN PATENT SPECIFICATION

(11) **EP 2 815 697 B1**
(45) Date of publication and mention of the grant of the patent: **11.09.2019**
(21) Application number: 12868512.0
(22) Date of filing: 13.02.2012
(51) Int. Cl.: A61B 5/02, A61B 8/02, A61B 8/06, A61B 5/1455, A61B 5/00

(54) **DEVICE FOR ASSESSING REGIONAL BLOOD CIRCULATION**
VORRICHTUNG ZUR BEURTEILUNG DER REGIONALEN BLUTZIRKULATION
DISPOSITIF D'ÉVALUATION DE LA CIRCULATION SANGUINE RÉGIONALE

(43) Date of publication of application: 24.12.2014
(73) Proprietor: Girina, Marina Borisovna, St.Petersburg 197343 (RU); Girin, Ivan Ivanovich, St.Petersburg 197343 (RU)
(72) Inventor: Girina, Marina Borisovna, St.Petersburg 197343 (RU); Girin, Ivan Ivanovich, St.Petersburg 197343 (RU)
(74) Representative: Sloboshanin, Sergej
(86) International application number: PCT/RU2012/000096
(87) International publication number: WO 2013/122495

(56) References cited:
- WO-A1-2010/076808
- RU-C1- 2 383 888
- RU-C1- 2 383 888
- RU-C2- 2 207 052
- US-A- 4 327 739
- US-A1- 2003 109 776
- US-A1- 2004 034 294
- US-A1- 2005 109 351
- US-A1- 2007 016 072
- US-A1- 2008 077 010
- US-A1- 2008 183 059
- US-A1- 2010 152 591
- US-A1- 2012 010 506
- US-B1- 6 468 219

## Description

### TECHNICAL FIELD

The present invention relates to a device for assessment of regional blood flow, tissue microcirculation and blood oxygen saturation according to claim 1. More generally, the invention belongs to the field of medicine, namely, to medical engineering and can be used for non-invasive (transcutaneous) and intraoperative (surgical) determination of parameters of blood flow and blood oxygen saturation in various fields of medicine: general medicine, beauty therapy, dermatology, traumatology, functional diagnostics, cardiology , neurosurgery, vascular surgery, urology, gynaecology, traumatic surgery, stomatology, maxillofacial surgery, plastic surgery, ophthalmology, rehabilitation, sports medicine, physiology, pathophysiology, pharmacology, oriental medicine.

### PRIOR ART

US6468219 B1 (NJEMANZE PHILIP CHIDI) discloses a device for assessment and display of regional blood flow, namely arterial blood flow, and blood oxygen saturation comprising a transcranial Doppler ultrasound device and an oximeter.

US 2008/077010 A1 (COHEN-SOLAL ERIC ET AL) discloses a device for assessment and display of blood flow and blood oxygen saturation comprising a Doppler transducer with distinct emitter and receivers elements used in combination with an oximeter.

To study tissue microcirculation in clinical practice the following methods are most frequently used: intravital microscopy, laser doppler flowmetry, radionuclide clearance method, transcutaneous pO2 and pCO2 determination, thermography. Each of them has its advantages and disadvantages, but none of them fully satisfies clinicians. The usual objects under study are vessels of the skin, mucous membrane and retina microvasculature. These methods have certain limitations concerning assessment of tissue microvasculature of internal organs, including intraoperative assessment.

There are Acuson-128XP and HP-1500 devices for peripheral vessels blood flow study (Ultrasound comprehensive study of patients with aortic aneurysm // Angiology and Vascular Surgery. -1996.-Nº 5.-P. 46-58). Already existing devices are equipped with 7.5 MHz operating frequency ultrasonic sensors.

There is also VASCULAB SP25A hardware system (Comparison of data received by means of lower limbs subcutaneous veins ultrasonic dopplerography and clinical varicose vein disease // Imaging in Hospital.-1996. - December. Nº 9. - P. 30-35). Subcutaneous vessels hemodynamics in the above hardware system is investigated with the help of an ultrasonic sensor with an operating frequency of 8 MHz in the CW Doppler mode. The ultrasonic beam is kept at a 45-50° angle in order to reduce errors while performing quantitative assessment of blood flow velocity.

The operating frequency of the above devices sensors is significantly less than 20 MHz, which does not allow for a clear signal yielding information about blood flow of a single micro vessel in 8-mm depth. Furthermore, the existing devices make it possible to evaluate the velocity of blood flow only without determining its direction which is necessary for early non-invasive diagnosis of various diseases, such as pulpitis, stomatitis, etc.

Another disadvantage of these devices is inability to conduct examination on-site and in a mobile hospital as these devices are bulky stationary units and before examining patients one requires an additional long-time preparation of devices in order to place them properly, connect cables of the electrodes and sensors.

The unit which possesses the most comparable technical characteristics and provides the most comparable results similar to the proposed device for assessment of regional blood flow, tissue microcirculation and blood oxygen saturation is the unit for assessment of regional blood flow comprising the device for blood flow measurement which includes a Doppler ultrasound transducer with a sensor equipped with receiving and emitting piezoelectric elements (RU, patent number 2152173, class A61V8/06, 1998).

The most comparable technical characteristics and results of the proposed method of assessment of regional blood flow, tissue microcirculation and blood oxygen saturation is the method of regional blood flow assessment through tissue location by means of ultrasonic Doppler sensor set at the sounding point and determination of blood flow characteristics by means of the data processing unit (RU, patent number 2152173, class A61V8/06, 1998).

The most significant disadvantage of the given method and device of regional blood flow evaluation is that it is impossible to carry out a comprehensive study of blood flow system of macro- and micro-vessels simultaneously with pulse oximeter parameters and also impossible to provide a comprehensive evaluation of the reaction demonstrated by the entire circulatory system to various physiological and functional tests.

Moreover, the given method and device have following shortcomings:
1. They cannot be used to determine velocity characteristics - linear and volumetric blood flow velocity in the tissues of a living organism during surgery as the design of the sensors the given device is equipped with does not provide for this function, and the sensors were used only transcutaneously (transdermally), it was impossible to use them intraoperatively because of their shape.
2. No option of desired signal detection amid self-noise during surgical interventions.
3. It is impossible to measure linear and volumetric blood flow velocity, oxygen saturation, heart rate (HR) simultaneously.
4. It is impossible to differentiate the signal, namely arteriolar, venular, capillary, shunt or lymph signal which otherwise ensures a higher level of general diagnostics.
5. It is impossible to determine oxygen saturation and pulse rate simultaneously with data on blood flow.
6. It is impossible to determine blood flow characteristics in a single slice and tissue section.

### DISCLOSURE OF INVENTION

The subject matter of the present invention is defined in claim 1, embodiments of which are subject of subclaims 2 and 3.

The technical result the present invention is aimed at is to develop a method and a mobile device for assessment of blood flow, tissue microcirculation and blood oxygen saturation, allowing precise control of linear and volumetric blood flow velocity, resistivity and pulse index, dopplerogram, plethysmogram and heart rate (HR).

The stated technical result is achieved due to the fact that design of the device for assessment of regional blood flow, tissue microcirculation and blood oxygen saturation, comprising a device for blood flow measurement which includes Doppler ultrasound transducer with a sensor comprising receiving and emitting piezoelectric elements, according to the claim, is enhanced with a device for pulse oximetry equipped with a sensor as well as the unit to determine the oximeter characteristics, the pulse oximeter sensor has two LEDs, one of which emits visible light in the red spectrum (600 nm), the other in the infrared spectrum (940 nm); moreover, the device for blood flow measurement includes two electronic software units to determine quantitative characteristics in a single vessel and in tissue section: macro-and micro-measurement modes; it should be noted that data from all the units are transmitted to the data display unit and the shape and design of the acoustic heads used in the sensors of blood flow measurement device correspond to the anatomic features of the region of interest: surgical, transcutaneous, laparoscopic, with the frequency of 10, 20, 25, 30 MHz, elongated, L-shaped; the sensors are enclosed in a titanium case for repeated sterilization conditions. Furthermore, the stated technical result is achieved due to the fact that the device for assessment of regional blood flow, tissue microcirculation and blood oxygen saturation, according to the claim, can have laparoscopic sensors used with 5- and 10-mm diameter trocars. Furthermore, the length of the acoustic heads of sensors installed in blood flow measurement device may be 0.1 ÷ 250mm, the sensor diameter is 1.5 ÷ 30mm and the L-shaped sensor angle is 135° with a radius of 10mm.

The stated technical result is achieved due to the fact that the method for assessment of regional blood flow, tissue microcirculation and blood oxygen saturation, through tissue location by means of the ultrasonic Doppler sensor set at the sounding point and through determination of blood flow characteristics by means of the data processing unit, according to the claim, the Doppler ultrasound transducer is set either into the projection of a single vessel or into the projection of or directly on the microcirculation volume of tissue; when working with a single vessel, Macro software and electronic unit is used which, while calculating, utilizes a special filter singling out the signal from a single vessel; and, when working with a microcirculation volume of tissue, Micro software and electronic unit is used which runs at maximum gain, while the pulse oximeter sensor is additionally set on the patient's ear or finger; from two LEDs of the pulse oximeter sensor, one of which emits visible light in the red spectrum (600 nm) and the other in the infrared spectrum (940 nm), the light passes through the tissue to a light detector with some portion of radiation absorbed by the blood and soft tissues, depending on the concentration of hemoglobin, the amount of the absorbed light at each of the wavelengths depends on the degree of hemoglobin oxygenation in the tissue; at the same time the pulse oximeter data processing unit singles out the blood pulse component from the absorption spectrum, i.e. it separates the arterial blood component from the venous or capillary blood permanent component, while the Doppler signal processing unit and the pulse oximeter data processing unit display data on the screen via the connected visualisation unit.

Furthermore, the stated technical result is achieved due to the fact that the method for assessment of regional blood flow, tissue microcirculation and blood oxygen saturation, according to the claim, presupposes that 25 MHz transcutaneous elongated sensor (cylindrical, the head length of 30 ± 2 mm, the effective diameter of 1.5 ± 0.2 mm) can be used in abdominal surgery, and the 10 MHz surgical sensor (cylindrical, the head length of at least 100 ± 5 mm, the effective diameter of 1.5 ± 0.2 mm), 20 MHz surgical curved sensor (cylindrical, the head length of 100 ± 5mm, the effective diameter of 1.5 ± 0.2 mm), 25 MHz surgical sensor (cylindrical, the head length of 100 ± 5 mm, the effective diameter of 1.5 ± 0.2 mm) can be used in neurosurgery.

20 MHz laparoscopic sensor (cylindrical, the head length of 250 ± 5 mm, the effective diameter of 1,5 ± 0.2 mm) is used in laparoscopic surgeries. In addition, 25 MHz L-shaped sensor (canted - the rounded working part of the head) is used to assess the irritating effects of substances on the vessels of chicken embryo chorioallantoic membrane (CAM).

The device for assessment of regional blood flow, tissue microcirculation and blood oxygen saturation is a universal diagnostic tool and can be used both in permanent hospital facilities and on-site, when examining astronauts immediately after landing, in sealed closed objects and in case of elevated pressure in decompression systems.

The method and device for assessment of regional blood flow, tissue microcirculation and blood oxygen saturation are of great scientific and clinical importance and help to detect pathological changes in tissue microcirculation in the early stages of development, they are also relevant for dynamic monitoring of recovery processes in the course of treatment. The method and device are in demand in research activities when studying the mechanisms of the cardiovascular system affected by various adverse factors.

This integrated approach allows one to evaluate changes in the blood circulatory system as early as at the stage of functional dysfunction.

### BRIEF DESCRIPTION OF THE VIEWS OF THE DRAWINGS

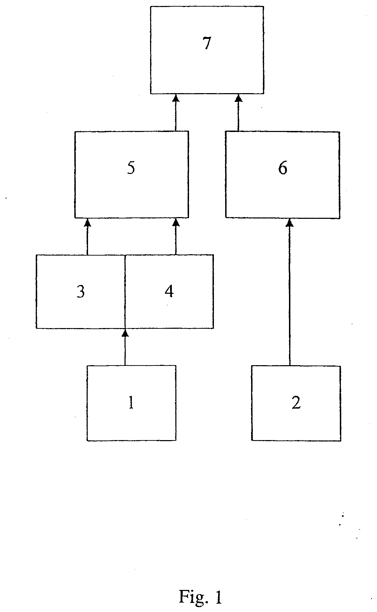

Figure 1 shows the device for assessment regional blood flow, tissue microcirculation and blood oxygen saturation.

The device for assessment of regional blood flow, tissue microcirculation and blood oxygen saturation includes a device for blood flow measurement (not shown in the drawing). The device includes a Doppler ultrasound transducer 1 with a sensor which comprises receiving and emitting piezoelectric elements (not shown in the drawing); a device for pulse oximetry with a sensor 2. The device for blood flow assessment includes two electronic and software units 3 and 4 to determine quantitative characteristics in a single vessel and tissue section: macro- and micro-measurement modes. Data from blocks 3 and 4 are transmitted to the display unit 5 (a Doppler signal data processing unit).

The shape and design of the acoustic heads used in the sensors of blood flow measurement device correspond to the anatomic features of the region of interest: surgical, transcutaneous, laparoscopic, with the frequency of 10, 20, 25, 30 MHz, elongated, L-shaped; the sensors are enclosed in a titanium case for repeated sterilization conditions.

The device for pulse oximetry includes unit 6 to determine pulse oximeter characteristics (pulse oximeter data processing unit).

The pulse oximeter sensor 2 has two LEDs (not shown in the drawing), one of which emits visible light in the red spectrum (600 nm), the other in the infrared spectrum (940 nm). All data is displayed on the visualisation unit 7.

The method for assessment of regional blood flow, tissue microcirculation and oxygen saturation implemented by the proposed device for assessment of regional blood flow, tissue microcirculation and oxygen saturation, is as follows:
The Doppler ultrasound transducer 1, with some gel applied thereto in advance, is set either into the projection of a single vessel or into the projection of or directly on the microcirculation volume of tissue. When working with a single vessel, Macro software and electronic unit 3 is used which, while calculating, utilizes a special filter singling out the signal from a single vessel. When working with a microcirculation volume of tissue, Micro software and electronic unit 4 is used which runs at maximum gain. During examination the pulse oximeter sensor 2 is additionally set on the patient's ear or finger, from two LEDs of the pulse oximeter sensor, one of which emits visible light in the red spectrum (600 nm) and the other in the infrared spectrum (940 nm), the light passes through the tissue to the light detector. Some portion of radiation is absorbed by the blood and soft tissues, depending on the concentration of haemoglobin, while the amount of the absorbed light at each of the wavelengths depends on the degree of haemoglobin oxygenation in the tissue. The pulse oximeter data processing unit 6 singles out the blood pulse component from the absorption spectrum, i.e. it separates the arterial blood component from the venous or capillary blood permanent component. At the same time the Doppler signal processing unit 5 and the pulse oximeter data processing unit 6 are connected with the visualisation unit 7 in order to display data on screen.

The Doppler ultrasound transducer 1 and the pulse oximeter sensor 2 are connected to the device in parallel. Data reading and processing are performed simultaneously.

During abdominal surgeries 25 MHz transcutaneous elongated sensor (cylindrical, the head length of 30 ± 2 mm, the working diameter of 1.5 ± 0.2 mm) is mostly used.

10 MHz surgical sensor (cylindrical, the head length of min. 100 ± 5 mm, the effective diameter of 1.5 ± 0.2 mm), 20 MHz surgical curved sensor (cylindrical, the head length of 100 ± 5 mm, the effective diameter of 1.5 ± 0.2 mm), 25 MHz surgical sensor (cylindrical, the head length of 100 ± 5 mm, the effective diameter of 1.5 ± 0.2 mm) can be used in neurosurgery. 20 MHz laparoscopic sensor (cylindrical, the head length of 250 ± 5 mm, the effective diameter of 1.5 ± 0.2 mm) is used in laparoscopic surgery. Furthermore, 25 MHz L-shaped sensor (canted - the rounded working part of the head) is used to assess the irritating effects of substances on the vessels of chicken embryo chorioallantoic membrane (CAM).

### EXAMPLES OF METHOD IMPLEMENTATION

### Neurosurgery:

The possibility to adequately assess various links of the cerebral blood flow and the blood oxygen saturation during neurosurgical interventions is crucial for successful surgical treatment in general. With the help of this device with an integrated oximeter one can adjust the plan and extent of surgeries, change the duration of certain procedures and regulate the degree of traction effects on the brain, thus reducing the risk of intra- and postoperative complications, as well as explore the possibility of assessment of blood flow in arteries and veins of different calibre in the cortex, brainstem, sinuses and cranial nerves arteries by means of surgical sensors with the frequency of 10, 20, 25 MHz.

### Example 1.

Intraoperative studies using the proposed device and method involved 70 patients with tumours and cerebrovascular pathology and were conducted by means of special sterilized sensors with the frequency of 10, 20 and 25 MHz. We investigated the possibility of assessing blood flow in the arteries and veins of different calibre in the cortex, brainstem, sinuses and cranial nerves arteries.

When studying large brain vessels such as medial cerebral artery branches, the adequate result was achieved by using a 20 MHz sensor, and made it possible to estimate the radical clipping of aneurysm, as well as the risk of a postoperative spasm.

During interventions in the region of the cranial sinuses, the use of a contact sensor enables one to evaluate the existing blood flow in the region of the sinus affected by a tumour tissue. The study of the arteries and veins of the cerebral cortex can be carried out with the help of 20-25 MHz sensor that can adequately assess velocity parameters of blood flow in vessels with the outer diameter of up to 0.25 mm.

We studied blood flow in small-diameter vessels of the cerebral cortex in patients with neuroectodermal tumours of different histological structure by means of functional loading tests. Preliminary evidence of a certain connection between the type of blood flow response to hyperventilation and the histological structure of the tumour has been obtained.

During the surgeries of tumours in the chiasmo-sellar region blood flow in the optic nerve was assessed and the criteria for prediction of possible impaired blood circulation in it were tried and tested.

It was found that the method and device for assessment of regional blood flow, tissue microcirculation and blood oxygen saturation enables assessment of blood flow in vessels of the brainstem and cranial nerves, including in the area of their immediate outlet from the brainstem (facial and vagus nerves). Thus, it is possible to conclude that the present method and device may be used in neurosurgery and provide some opportunities to control blood flow in different vessels of the brain and cranial nerves.

### Example 2.

The study involved 12 patients with pituitary adenomas and meningiomas of the tubercle of sella turcica - three men and nine women. The patients were aged 42- 69. 4 patients were diagnosed with meningioma of the tubercle of sella turcica and 8 patients were diagnosed with pituitary adenoma. The clinical presentation is predominantly visual malfunctions and (in case of adenomas) hormonal disorders of varying intensity.

All patients underwent a surgery - bifrontal trepanation, removal of the tumor. Various parts of the sagittal sinus were located before and after opening the dura mater and also under the conditions of hyperventilation by means of 20 MHz ultrasonic sensor. Blood flow through the optic nerves arteries was monitored while accessing the tumor immediately after the optic nerves were detected by means of the same device with the help of 25 MHz micro-Doppler sensor.

Findings: parameters of the linear velocity of blood flow in the initial sections of the anterior third of the superior sagittal sinus are 6 ± 2.1 cm/sec maximum and 4 ± 1.8 cm/sec minimum. Velocity characteristics increase toward the sinus distal parts and on the border of the anterior and middle thirds are as high as 18 ± 6.6 cm/sec maximum and 9 ± 4.4 cm/sec minimum.

When opening the dura mater (up to the moment the beginning of the sinus is separated from the cockscomb), it was noted that velocity values decrease by 70-90% in the initial areas of the sinus and by 20- 50% on the border of the anterior and middle thirds.

Blood flow through the optic nerves arteries was monitored at the exact moment when accessing the tumor under direct vision. The average speed through the optic nerves arteries was 24.5 ± 4.8 cm/sec, PI - 0,67 ± 0.11, RI - 0.49 ± 0.07.

The operated patients showed different dynamics of visual functions: three patients manifested improved vision (acuity, field) after the surgery, three patients did not manifest any dynamics and two patients had impairment of vision.

Both patients with consequent impairment of vision showed dramatically increased pulse index (1.1 and 1.3, respectively), as well as, to a lesser extent, increased resistive index (0.6 and 0.8, respectively), with medium-velocity characteristics (1 and 24 cm/sec) preserved.

Thus, we can assume the possibility of intraoperative prediction of the state of blood flow in the optic nerve, with further correction of the scope of manipulations in the optic nerve.

### Use in cavity and laparoscopic surgery

Ultrasonic dopplerography together with a pulse oximeter can be used in various fields of surgery to determine microcirculatory blood flow and hemodynamics in major vessels of various diameters. Possible areas of application:
- studies conducted in the intestine during surgery for acute intestinal failure: it is possible to determine indicators of blood flow and blood oxygen saturation after reduction of incarcerated intestine in case of strangulation, in the borders of the resected intestine before application of anastomosis, assessment of intestinal viability in case of strangulated and incarcerated hernia;
- in vascular surgery: intraoperative assessment of blood flow and assessment of blood oxygen saturation in blood vessels immediately after revascularization;
- assessment of blood flow in flaps and transplants, including those used in the esophagus plastic procedures, determination of blood flow and blood oxygen saturation in the large intestine (descendent colon) in case of abdominal-anal resection.

### Example 3.

At present myocardial revascularization (coronary artery bypass grafting - CABG) in patients with ischemic heart disease has become a conventional method of treatment of this disease. The effectiveness of surgical treatment of ischemic heart disease (IHD) is determined by many factors - the patient's age, concomitant diseases (old myocardial infarction, left ventricular aneurysm, hypertension, etc.) and, primarily, the extent of coronary arteries and myocardial microvasculature damage. Considering the above, the key issue in improving the results of surgical treatment of patients with ischemic heart disease (IHD) remains an objective assessment of the coronary and myocardial blood flow prior to the main stage of intervention and after revascularization surgery.

Modern methods dealing with recording of blood flow in the coronary arteries and assessment of total blood flow in the myocardial microvasculature which are introduced in cardiac surgery practice enable one to find new ways of tackling this problem.

Objective: to evaluate the possibility of use of high-frequency ultrasound Doppler flowmetry device for intraoperative monitoring and assessment of blood flow in the coronary arteries and total blood flow in the microvasculature section of myocardium.

### Parameters measured by the device:

### Indexes:

Ri - total peripheral resistance index (Purcell factor). RI = (Vs-Vd)/Vs
Vs - maximum systolic velocity.
Vd - end-diastolic velocity.
ISD - systolic and diastolic index (Stewart index) reflects the elastic properties of blood vessels. ISD = Vs/Vd/
Pi - pulsatility index (Gosling index) reflects the elastic properties of the arteries. Pi = (Vs-Vd)/Vm.

### Blood flow linear characteristics:

Vas - maximum systolic velocity on medium velocity curve.
Vam - average velocity on the medium velocity curve.
Vd - maximum diastolic velocity on medium velocity curve.

### Blood flow volumetric characteristics:

Qas - maximum systolic velocity on medium velocity curve.

### Stages of the study:

The study was performed intraoperatively with all of the above blood flow characteristics being recorded. Myocardial blood flow was investigated in the apex of heart area on the myocardium of the left and right ventricles.

The study was performed in accordance with the stages of the surgery. Stage 1 - measurement of blood flow characteristics prior to cardiopulmonary bypass, i.e. after pericardiotomy the surgeon placed a sterile ultrasound transducer with a frequency of 20 MHz ultrasonic vibrations at the angle of 60° into the projection of the coronary artery. The sensor with a frequency of 25 MHz ultrasonic vibrations was placed directly on the myocardium in the apex of heart area. Stage 2 - during cardioplegia the sensor with an operating frequency of 25 MHz was placed directly into the myocardium. Stage 3 - after anastomoses were performed and blood flow was re-established, there was assessment of linear and volumetric characteristics of blood flow in the myocardium, through the graft, in the area of the distal anastomoses and in the coronary arteries distal of the graft.

During all stages of the surgery peculiarities of blood flow were determined in the ramus interventricularis anterior (RIVA), right coronary artery (RCA) and circumflex branch (RCX). The criteria for selection of patients for the study was manifested lesion of the arteries.

For each artery sensors with a certain operating frequency were selected: RIVA, RCX, RCA - 10 MHz; in the distal end of RIVA - 20 MHz; directly on the myocardium - 25 MHz.

All measurements were performed in the supine position at a constant temperature and humidity. All patients had their arteriolar pressure and heart rate measured on-line.

### Details of clinical impressions:

The study included 21 patients of both sexes. All patients were diagnosed with ischemic heart disease (IHD) and cardiac angina functional class 3-4. To evaluate the effectiveness of the method the patients were divided into 3 groups: group I - normothermic cardiopulmonary bypass with normothermic blood cardioplegia; group II - hypothermic cardiopulmonary bypass with Custodiol cardioplegia; group III - surgeries without cardiopulmonary bypass. With the purpose of monitoring all the patients had echocardiography done in the pre- and postoperative periods. All studies were carried out during coronary artery bypass surgery.

### Results of the study:

Peculiarities of coronary blood flow when operating on the patients of group I.

When analysing the patients of group I at the beginning of the surgery, the total BFLV (blood flow linear velocity) value in RIVA, DB, RCA, PIV (posterior interventricular artery) and in the myocardium decreased from 46 ± 5 cm/s to 39 ± 2 cm/s before cardiopulmonary bypass was started (p> 0.05). At the stage of distal anastomoses no significant decrease in BFLV values was recorded as compared to the beginning of normothermic cardiopulmonary bypass (p> 0.05). After the clamp was removed from the aorta there was a rapid increase in BFLV in LCA and RCA circulation up to 62 ± 4 cm/s as compared with the stage of distal anastomoses (p <0.05). BFLV remained at this level until the end of the surgery, which is related to adequate coronary blood flow restoration.

A similar pattern was observed when analyzing tissue myocardial microcirculation in the projection of RIVA and RCA at the beginning and at the end of the surgery with physiological decline in average blood flow velocity. Thus, at the beginning of the surgery the total values of velocity were decreasing from 43 ± 3 cm/s to 36 ± 4 cm/s before cardiopulmonary bypass was started (p> 0.005). At the stage of distal anastomoses there was no record of significant decrease in velocity as compared with the beginning of normothermic cardiopulmonary bypass (p> 0.05). After the clamp was removed from the aorta, velocity was rapidly increased in RIVA and RCA circulation up to 50 ± 6 cm/s as compared with the stage of distal anastomoses (p<0.05) which remained at this level until the end of the surgery.

The study of BFLV and tissue microcirculation in LV (left ventricle) and RV (right ventricle) in group I revealed the fact of steady blood supply in RIVA, RCA, the myocardium of LV and RV during the whole surgery. This is related to perfusion of the coronary bed with normothermic oxygenated blood cardioplegic mixture at the stage of aortic clamping with minimal demand in oxygen and energy substrate because of asystole.

Peculiarities of coronary blood flow when operating on the patients of group II.

When analyzing the patients of group II, BFLV values were decreased from 45 ± 5 cm/sec to 0 at the stage of aortic clamping which corresponds to the period of myocardial ischemia (p <0,05). Removal of the clamp from the aorta led to restored BFLV in LCA and RCA circulation up to 59 ± 7 cm/s, which remained at this level until the end of the surgery.

When analyzing tissue microcirculation, one could observed decrease in average blood flow velocity in the myocardium from 41 ± 4 cm/sec to 0 prior to the stage of distal anastomoses (p <0.05), followed by a rapid increase to 47 ± 6 cm/sec after the clamp was removed from the aorta.

Complete termination of coronary blood flow at the stage of aortic clamping in group III, a rapid increase in linear and volumetric characteristics of coronary blood flow, as well as increased perfusion of myocardial microcirculation after bypass surgery and blood flow restoration caused reperfusion injuries during the transition from anaerobic to aerobic metabolism and from hypo- to normothermic blood circulation.

Peculiarities of coronary blood flow when operating on the patients of group III.

When analysing the patients of group III at the beginning of the surgery, the total BFLV value in RIVA, DB, RCA, PIV and in the decreased from 48 ± 4 cm/s to 41 ± 2 cm/s prior to the stage of distal anastomoses due to myocardial stabilization devices (p> 0.05). At the stage of distal anastomoses no significant decrease in BFLV values was recorded as compared to the beginning of the surgery. After adequate coronary blood flow was restored, as compared to the stage of distal anastomoses, there was a rapid increase in BFLV in LCA and RCA circulation up to 63 ± 5 cm/s (p <0.05) which remained at this level until the end of the surgery.

A similar pattern was observed when analyzing tissue myocardial microcirculation at the beginning and at the end of the surgery with physiological decline in average blood flow velocity. Thus, at the beginning of the surgery the total value of velocity was decreasing from 44 ± 3 cm/s to 39 ± 2 cm/s prior to the stage of distal anastomoses (p> 0.005). As compared to the stage of distal anastomoses, after it was completed, velocity was rapidly increased in LCA and RCA circulation up to 53 ± 4 cm/s and remained at this level until the end of the surgery. The parameters of tissue microcirculation in group III were favourable for bypass surgery on RCA and RIVA circulation. When performing bypass surgery on the posterolateral artery (PLA) and obtuse marginal artery there was a significant decrease in linear and volumetric blood flow characteristics, leading to impaired tissue microcirculation associated with increased demand in oxygen and energy substrates. It is caused by significant impairment of intracardiac hemodynamics when dislocating the heart and providing access to the coronary arteries on its posterolateral surface.

The conducted high-frequency ultrasound Doppler flowmetry monitoring of coronary blood flow during cardiac surgeries showed that an important stage of the surgery is insersion of an aortic cannula. In the course of uncomplicated cannulation of the ascending aorta there was no microembolia recorded. Increased duration of manipulations with the aorta resulted in microembolia in LCA and RCA circulation. The beginning of cardiopulmonary bypass was always accompanied by changes in blood flow velocities and decreased myocardial microvasculature perfusion. However, in all cases the average linear flow velocity exceeded its critical value (20 cm/s). Preserved autoregulation of coronary blood flow is likely to compensate for short-term episodes of decrease in arteriolar pressure.

Complete cardiopulmonary bypass significantly changed the shape of the dopplerogram - blood flow acquired a "non-pulsating" sinusoidal character. At the stage of aortic clamping there was no coronary blood flow in group II. After cardiac function is restored and blood flow is released, BFLV values and myocardial microcirculation are restored to their original values. However, group I manifested a greater decrease in myocardial tissue microcirculation in the affected artery circulation.

Significantly high linear and volumetric characteristics of coronary blood flow and blood flow through the graft, as well as a significant increase in myocardial microcirculation perfusion during performance of distal anastomoses were recorded in groups I and III as compared to group II. At the same time, there were no considerable differences between groups I and III. Cardioplegic mixture in group I and blood in group III were evenly supplied to both LV and RV through the system of RIVA, circumflex branch and PCA throughout the surgery, which excluded the period of myocardial ischemia.

The conducted high-frequency ultrasound Doppler flowmetry monitoring of coronary blood flow and myocardial perfusion during cardiac surgeries with cardiopulmonary bypass showed that during hypothermic cardiopulmonary bypass there was a steady tendency of decreased blood flow and perfusion in the microcirculation area, the pulsating character of perfusion changed into sinusoidal. It should be noted that these changes affected hypothermic perfusion to a greater extent, they were observed less often during normothermic cardiopulmonary bypass, and least often during surgeries without CPB cardiopulmonary bypass. The method allowed not only to evaluate the characteristics of coronary blood flow but also the adequacy of perfusion restorationy in microcirculation area of the coronary bed after myocardial revascularization. The study showed that the impaired coronary bed causes the asymmetry of blood flow in LV and RV, reduced BFLV in the affected artery circulation and a compensatory increase of BFLC in the unaffected artery circulation. The same regularity is observed in the coronary bed microcirculation. This regularity was recorded in all investigated groups.

In order to illustrate the above, we took the ratio of the velocity of total microcirculatory blood flow in the myocardium of the ventricles (myocardium of the left ventricular/ myocardium of the right ventricular - LV/RV). So, it was observed that there is homogeneous distribution of blood-base normothermic cardioplegic solution between the myocardium of the left and right ventricles of the heart, as evidenced by the values registered during surgery - LV/RV = 1. In case of pharmaco-hypothermic cardioplegia these values were LV/RV = 1,25 (p <0,02).

One of the consequences of prolonged perfusion of the heart is development of myocardial stunning manifested by the average blood flow linear velocity increased by more than two standard deviations from the baseline together with a reduced peripheral resistance. We did not record any case of myocardial stunning syndrome affecting the patients, which was confirmed by transesophageal echocardiography before the surgery and on the operating table after cardiac function and blood flow were restored. This shows the effectiveness of all analyzed methods despite the period of ischemia and reperfusion injuries in group II.

Thus, the given device is an explicit and highly informative method for intraoperative assessment of coronary and myocardial blood flow.

### Potential use in hygiene, toxicology and sanitary science

25 MHz L-shaped sensor (canted - the rounded working part of the head) is used for toxicological research and testing of perfume and cosmetics (PC) products, oral care products, as well as raw materials for their production, including those obtained with the use of nanotechnology or containing nanomaterials.

With the help of this device one can perform quantitative assessment of blood flow velocity in case of irritation.

### Example 4.

The egg is placed on the fixing stand with its blunt end upwards and then the air cell is opened. Shell is removed from the whole air cell, then the surface of the outer shell membrane is moistened with normal saline solution and it is completely removed so as not to damage the chorioallantoic membrane (CAM). CAM is covered with layers of 400 mm³ of normal saline solution (to prevent it from drying) The egg is placed in a temperature-controlled chamber for 20 min to eliminate the traumatic shock. (If there is some damage to CAM or bleeding occurs, the egg is rejected).

Having selected a blood vessel on the surface of CAM, the sensor is positioned at 60° to the examined vessel, while ensuring dopplerogram with low amplitude pulsations and no sharp peaks (the slowly varying wave) which are displayed on the screen of the device.

After blood flow velocity in the selected vessel is measured in minute 0, the extract of the tested sample is applied in layers in the amount of 400 mm³. Minute 0 corresponds to the intact condition of the embryo CAM vessels. After 80 seconds the change in blood flow velocity in CAM vessels under the influence of the tested sample is evaluated.

At least 10 embryos should be analysed in order to test one sample.

The software developed for the device analyzes the dopplerogram automatically by calculating blood flow linear velocity (Vs parameter, cm/sec). The software allows one to single out tissue microsections filled with arterial, venular, capillary and bypass blood flow. The following histogram is introduced: 4 columns display % of particles moving at an appropriate speed in ascending order. The calculation of K ratio was introduced. K ratio is % of capillary blood flow in the given tissue section. In order to perform calculations an additional filter was installed which cuts off all velocities exceeding 0.1 cm/s.

The four-channel feature of the device allows to register micro- and macro-signals simultaneously in real time at 4 points.

Due to electronic, software and hardware and mechanical design of the sensors combined, taking into account additional filtering, one can measure low velocity starting from 0.001 cm/s.

### INDUSTRIAL APPLICABILITY

Thus, the use of the proposed device for assessment of regional blood flow, tissue microcirculation and blood oxygen saturation and the proposed method for assessment of regional blood flow, tissue microcirculation and blood oxygen saturation allows precise control of linear and volumetric blood flow velocity, resistivity and pulse index, dopplerogram, plethysmogram and heart rate (HR).

## Claims

1. Device for assessment of regional blood flow, tissue microcirculation and blood oxygen saturation, comprising
- a device for blood flow measurement which includes a Doppler ultrasound transducer (1) with a first sensor having an acoustic head, wherein the first sensor comprises distinct receiving and emitting piezoelectric elements,
- an apparatus for pulse oximetry equipped with a second sensor (2) and a pulse oximeter processing unit (6) to determine blood oxygen saturation,
wherein the second sensor (2) has two LEDs, one of which emits visible light in the red spectrum, the other in the infrared spectrum,
wherein the device for blood flow measurement includes a first electronic-software unit (3) and a second electronic-software unit (4) for determining quantitative blood flow characteristics in a single vessel and in microcirculation volume of tissue, respectively, wherein the first electronic-software unit (3) comprises a filter adapted to single out the signal from a single vessel which is possible when the transducer (1) is set into the projection of a single vessel, wherein the second electronic-software unit (4) is adapted to work at a higher gain than the first electronic-software unit (3) to extract a tissue microcirculation signal which is possible when the transducer (1) is set on the microcirculation volume of a tissue,
wherein data from the first and the second electronic-software units (3, 4) are transmitted to a Doppler signal processing data unit (5),
wherein the shape and structure of the acoustic head of the first sensor is configured:
- to be elongated or L-shaped,
- to operate with at least one of the frequencies 10, 20, 25, 30 MHz,
- to be enclosed in a titanium case for providing repeated sterilization conditions,
wherein the Doppler ultrasound transducer (1) and the apparatus for pulse oximetry with the second sensor (2) are connected to the device for assessment of regional blood flow, tissue microcirculation and blood oxygen saturation in parallel to perform data reading and processing simultaneously,
wherein the Doppler signal processing unit (5) and the pulse oximeter data processing unit (6) are connected with a visualisation unit (7) configured to display on screen blood oxygen saturation data from the pulse oximeter unit (6), and the blood flow data in the single vessel or in the microcirculation volume of tissue from the Doppler signal processing unit (5).

2. The device according to claim 1, **characterized in that** laparoscopic sensors are used with trocars of 5 mm and 10 mm in diameter.

3. The device according to claim 1, **characterized in that** the length of the acoustic heads of the sensors of the blood flow measurement device is 0.1 - 250 mm, the diameter of the sensor is 1.5 - 30 mm and the angle of L-shaped sensor is 135° with the radius of 10 mm.

## Patentansprüche

1. Vorrichtung zur Auswertung der regionalen Blutzirkulation, Gewebemikrozirkulation und Sauerstoffsättigung im Blut, aufweisend
- eine Vorrichtung zur Messung des Blutflusses, die einen Doppler-Ultraschallwandler (1) umfasst mit einem ersten Sensor, der einen Akustikkopf hat, wobei der erste Sensor unterschiedliche piezoelektrische Empfangs- und Sendeelemente aufweist,
- ein Gerät zur Pulsoximetrie ausgestattet mit einem zweiten Sensor (2) und einer Pulsoximeterverarbeitungseinheit (6) zur Bestimmung der Sauerstoffsättigung im Blut,
wobei der zweite Sensor (2) zwei LEDs hat, von denen die eine sichtbares Licht im roten Spektrum emittiert, und die andere im Infrarotspektrum,
wobei die Vorrichtung zur Messung des Blutflusses eine erste Elektronik-Software-Einheit (3) und eine zweite Elektronik-Software-Einheit (4) zur Bestimmung von quantitativen Blutflusscharakteristika in einem einzelnen Gefäß bzw. in einem Gewebemikrozirkulationsvolumen umfasst, wobei die erste Elektronik-Software-Einheit (3) einen Filter zur Aussonderung des Signals von einem einzelnen Gefäß umfasst, das möglich ist wenn der Wandler (1) in die Projektion eines einzelnen Gefäßes gesetzt ist, wobei die zweite Elektronik-Software-Einheit (4) dazu vorgesehen ist, mit einer höheren Verstärkung als die erste Elektronik-Software-Einheit (3) zu arbeiten zur Aussonderung eines Signals der Gewebemikrozirkulation, das möglich ist wenn der Wandler (1) auf das Gewebemikrozirkulationsvolumen gesetzt ist,
wobei die Daten der ersten und der zweiten Elektronik-Software-Einheit (3, 4) zu einer Doppler-Signalverarbeitungseinheit (5) übertragen werden,
wobei die Form und der Aufbau des Akustikkopfes des ersten Sensors dazu vorgesehen sind:
- länglich oder in L-Form zu sein,
- mit wenigstens einer der Frequenzen von 10, 20, 25, 30 MHz zu arbeiten,
- in einem Titangehäuse eingeschlossen zu sein, um wiederholt sterile Bedingungen zu gewährleisten,
wobei der Doppler-Ultraschallwandler (1) und das Gerät zur Pulsoximetrie mit dem zweiten Sensor (2) mit der Vorrichtung zur Auswertung der regionalen Blutzirkulation, Gewebemikrozirkulation und Sauerstoffsättigung im Blut parallel verbunden sind, um das Auslesen und Verarbeiten von Daten gleichzeitig auszuführen,
wobei die Doppler-Signalverarbeitungseinheit (5) und die Pulsoximeterverarbeitungseinheit (6) mit einer Visualisierungseinheit (7) verbunden sind, die zur Anzeige der Daten der Sauerstoffsättigung im Blut von der Pulsoximetereinheit (6) und der Daten des Blutflusses in dem einzelnen Gefäß oder in dem Gewebemikrozirkulationsvolumen von der Doppler-Signalverarbeitungseinheit (5) auf einem Bildschirm ausgelegt ist.

2. Vorrichtung nach Anspruch 1, **dadurch gekennzeichnet, dass** laparoskopische Sensoren mit Trokaren von 5 mm und 10 mm im Durchmesser eingesetzt werden.

3. Vorrichtung nach Anspruch 1, **dadurch gekennzeichnet, dass** die Länge der Akusticköpfe der Sensoren der Vorrichtung zur Messung des Blutflusses 0,1 - 250 mm ist, der Durchmesser des Sensors 1,5 - 30 mm ist und der Winkel des L-förmigen Sensors 135° ist mit einem Radius von 10 mm.

## Revendications

1. Dispositif d'évaluation du débit sanguin régional, de la microcirculation tissulaire et de la saturation en oxygène du sang, comprenant
- un dispositif de mesure de débit sanguin qui comporte un transducteur ultrasonore Doppler (1) avec un premier capteur ayant une tête acoustique, où le premier capteur comprend des éléments piézoélectriques de réception et d'émission distincts,
- un appareil d'oxymétrie de pouls équipé d'un deuxième capteur (2) et d'une unité (6) de traitement d'oxymètre de pouls pour déterminer la saturation en oxygène du sang,
dans lequel le deuxième capteur (2) a deux LED, dont l'une émet une lumière visible dans le spectre rouge, l'autre dans le spectre infrarouge,
dans lequel le dispositif de mesure de débit sanguin comporte une première unité logicielle électronique (3) et une deuxième unité logicielle électronique (4) pour déterminer des caractéristiques quantitatives de débit sanguin dans un vaisseau unique et dans un volume de microcirculation de tissu, respectivement, où la première unité logicielle électronique (3) comprend un filtre adapté pour isoler le signal d'un vaisseau unique, ce qui est possible lorsque le transducteur (1) est placé dans la projection d'un vaisseau unique, où la deuxième unité logicielle électronique (4) est adaptée pour fonctionner à un gain supérieur à celui de la première unité logicielle électronique (3) pour extraire un signal de microcirculation tissulaire, ce qui est possible lorsque le transducteur (1) est placé sur le volume de microcirculation d'un tissu,
dans lequel des données provenant des première et deuxième unités logicielles électroniques (3, 4) sont transmises à une unité (5) de traitement de données de signal Doppler,
dans lequel la forme et la structure de la tête acoustique du premier capteur sont configurées :
- pour être allongées ou en forme de L,
- pour fonctionner avec au moins une des fréquences 10, 20, 25, 30 MHz,
- pour être enfermées dans un boîtier en titane pour fournir des conditions de stérilisation répétées,
dans lequel le transducteur ultrasonore Doppler (1) et l'appareil d'oxymétrie de pouls avec le deuxième capteur (2) sont connectés au dispositif d'évaluation du débit sanguin régional, de la microcirculation tissulaire et de la saturation en oxygène du sang en parallèle pour effectuer une lecture et un traitement de données simultanément,
dans lequel l'unité (5) de traitement de signal Doppler et l'unité (6) de traitement de données d'oxymètre de pouls sont connectées à une unité de visualisation (7) configurée pour afficher sur un écran des données de saturation en oxygène du sang provenant de l'unité (6) d'oxymètre de pouls, et les données de débit sanguin dans le vaisseau unique ou dans le volume de microcirculation de tissu provenant de l'unité (5) de traitement de signal Doppler.

2. Dispositif selon la revendication 1, **caractérisé en ce que** des capteurs laparoscopiques sont utilisés avec des trocarts de 5 mm et de 10 mm de diamètre.

3. Dispositif selon la revendication 1, **caractérisé en ce que** la longueur des têtes acoustiques des capteurs du dispositif de mesure de débit sanguin est de 0,1 à 250 mm, le diamètre du capteur est de 1,5 à 30 mm et l'angle du capteur en forme de L est de 135° avec le rayon de 10 mm.
